# EUROPÄISCHE PATENTANMELDUNG

(11) **EP 1 872 779 A1**
(43) Veröffentlichungstag der Anmeldung: **02.01.2008**
(21) Anmeldenummer: 06013376.6
(22) Anmeldetag: 28.06.2006
(51) Int. Cl.: A61K 9/50, A61K 31/55, A61P 25/24

(54) **Stabile multipartikuläre pharmazeutische Zusammensetzung von tri- und tetracyclischen Antidepressiva**

(71) Anmelder: Oramon Arzneimittel GmbH, 88471 Laupheim (DE)
(72) Erfinder: Braun, Michael, Dr., 89250 Senden (DE); Heinz, Carmen, 88477 Schwendi (DE); Beckert, Thomas, Dr., 88447 Birkenhard (DE)
(74) Vertreter: UEXKÜLL & STOLBERG

(57) **Zusammenfassung**

Die Erfindung betrifft eine multipartikuläre pharmazeutische Zusammensetzung, die Teilchen, die ein tri- oder tetracyclisches Antidepressivum oder ein pharmazeutisch annehmbares Salz oder Solvat davon und ein spezielles Bindemittel enthalten, und eine Schicht auf den Teilchen, welche eine Mischung von Ethylcellulose und Hydroxypropylmethylcellulose als verzögert freisetzendes Polymer enthält, aufweist.

## Beschreibung

Die vorliegende Erfindung betrifft eine multipartikuläre pharmazeutische Zusammensetzung, die ein tri- oder tetracyclisches Antidepressivum oder ein pharmazeutisch annehmbares Salz oder Solvat davon enthält, sowie ein Verfahren zu deren Herstellung.

Tri- und tetracyclische Antidepressiva werden seit längerem erfolgreich in der Therapie oder Linderung der als "depressives Syndrom" bezeichneten psychischen, somatischen oder psychosozialen Symptome verwendet.

Zu den tricyclischen Antidepressiva zählen neben dem bekanntesten Vertreter Amitriptylin auch Amitriptylinoxid, Clomipramin, Desipramin, Dioxepin, Ipramin, Lofepramin, Melitracen, Nortriptylin, Noxiptilin, Protriptylin sowie Trimipramin. Beispiele für tetracyclische Antidepressiva sind u.a. Maprotilin, Mianserin, Nomifensin.

Von Amitriptylin wird gegenwärtig das Hydrochloridsalz als herkömmliche schnell freisetzende Tablette oder als verzögert freisetzende Arzneiform verabreicht. Die verzögert freisetzenden Zusammensetzungen sind in Form einer teilbaren Hydrokolloidmatrixtablette oder als mit Retardpellets befüllte Hartkapseln erhältlich.

Es ist bekannt, dass multipartikuläre Darreichungsformen, wie mit Retardpellets befüllte Hartkapseln, im Vergleich zu monolithischen Arzneiformen, wie Matrixtabletten, zahlreiche Vorteile bieten. Viele dieser Vorteile leiten sich aus dem Verhalten von multipartikulären Arzneiformen im GastroIntestinaltrakt ab. Durch ihre geringe Größe können kleinere Formkörper wie Pellets den Magen unabhängig von dessen Füllungszustand und Motilität passieren. Im Gegensatz dazu kann es für größere Formkörper wie monolithische Tabletten zu stark schwankenden Transitzeiten im Magen kommen, da der Pylorus diese größeren Partikel nur in bestimmten Phasen der Magenperistaltik und in Abhängigkeit vom Füllzustand des Magens passieren lässt. Starke Schwankungen in der Magenpassagezeit führen jedoch zu unerwünscht hohen Streuungen in der Pharmakokinetik und Pharmakodynamik der Wirkstoffe. Aufgrund der gleichmäßigeren Passagezeiten gegenüber monolithischen Arzneiformen können mit multipartikulären Formulierungen somit reproduzierbarere inter- und intraindividuelle Plasmaverläufe erzielt werden.

Zudem ist bei multipartikulären Formen das Risiko des plötzlichen Versagens der modifizierten Arzneistofffreisetzung geringer, da die Einzeldosis auf viele Partikel aufgeteilt ist. Dagegen können bei monolithischen Retardpräparaten beispielsweise Risse in dem retardierenden Überzug zu einem plötzlichen Freisetzen der gesamten Dosis führen (dose dumping). Außerdem ist durch die gleichmäßigere Verteilung von multipartikulären Arzneiformen im Magen-Darm-Trakt das Risiko einer Irritation der Schleimhäute durch hohe lokale Konzentrationen herabgesetzt. Des Weiteren ist der Wirkstoff bei multipartikulären Präparaten in der sich an den Magen anschließenden Darmpassage über ein größeres Volumen verteilt, was sich ebenfalls vorteilhaft auf die Bioverfügbarkeit auswirken kann. Falls das Schlucken einer Kapsel oder Tablette für den Patienten nicht möglich ist, können kleinere Formkörper wie Pellets mit etwas Flüssigkeit auch getrunken oder über eine Ernährungssonde verabreicht werden.

Bei den tri- und tetracyclischen Antidepressiva handelt es sich um substituierte Diphenylamin- und Diphenylmethanderivate, in denen die Phenylsubstituenten zweifach verbrückt sind, sodass die Ringsysteme stark gewinkelte Strukturen aufweisen. Auf Grund der stark gewinkelten Strukturen neigen alle tri- und tertacyclischen Antidepressiva, so auch Amitriptylin, zur Zersetzung, insbesondere bei der Einwirkung von Feuchtigkeit und/oder erhöhten Temperaturen. Die Zersetzungsprodukte sind in fertigen Arzneiformulierungen unerwünscht, weshalb die europäische Pharmakopoe maximale Gehalte an Zersetzungsproduken vorschreibt.

Weiterhin kann es auch zu Wechselwirkungen mit der die Wirkstofffreisetzung steuernden Diffusionsmembran kommen, was zu Veränderungen der Wirkstofffreisetzung führt. Insbesondere die eigentlich wünschenswerten multipartikulären Amitriptylinzusammensetzungen haben sich in der Vergangenheit als lagerinstabil hinsichtlich Wirkstoffdegradation und -freisetzung erwiesen und wurden daher zum Teil durch Hydrokolloidtabletten ersetzt.

Um stabile multipartikuläre, vorzugsweise retardierte Arzneiformen von tricyclischen Antidepressiva wie Amitriptylin bereitzustellen, offenbart die EP 1 424 068 ein Verfahren, bei dem Neutralpellets vorgelegt werden, Amitriptylin unter gleichzeitigem Aufsprühen einer wässrigen Schellacklösung ohne Zuluft eingestreut wird und ggf. anschließend weitere Schichten wie Isolierschicht und Retardlack aufgebracht werden. Die Wirkstoffschicht kann neben Amitriptylin und wasserlöslichem Schellack weiterhin einen Zucker und/oder ein Antioxidans enthalten. Mit Hilfe dieses Verfahrens konnten Pellets erhalten werden, die eine verbesserte chemische Wirkstoffstabilität aufweisen. Unerwünschterweise verändert sich jedoch das Freisetzungsprofil des Wirkstoffs in diesen Zusammensetzungen im Verlaufe der Lagerung.

Der vorliegenden Erfindung liegt daher die Aufgabe zu Grunde, eine multipartikuläre pharmazeutische Zusammensetzung von tri- und tetracyclischen Antidepressiva, insbesondere Amitriptylin, zur Verfügung zu stellen, die die zuvor erwähnten Probleme vermeidet. Insbesondere sollte der in einer solchen Zusammensetzung enthaltene Wirkstoff während der Lagerung chemisch stabil sein und sich das Wirkstofffreisetzungsprofil während der Lagerung gegenüber dem ursprünglichen Freisetzungsprofil nicht oder nur geringfügig ändern, d.h. die Zusammensetzung sollte lagerstabil hinsichtlich der Wirkstoffdegradation und - freisetzung sein.

Diese Aufgaben werden durch die pharmazeutische Zusammensetzung gemäß Ansprüchen 1 bis 20 gelöst. Die Erfindung betrifft auch das Verfahren zu deren Herstellung gemäß den Ansprüchen 21 und 22.

Die erfindungsgemäße multipartikuläre pharmazeutische Zusammensetzung weist
(a) Teilchen, die
   (a1) ein tri- oder tetracyclisches Antidepressiva oder ein pharmazeutisch annehmbares Salz oder Solvat davon und
   (a2) ein Bindemittel ausgewählt aus Hydroxypropylmethylcellulose, Hydroxypropylcellulose, Polyvinylpyrrolidon, Polyvinylalkohol, Methylcellulose, Natriumcarboxymethylcellulose oder Mischungen davon
   enthalten, und
(b) eine Schicht auf den Teilchen (a), welche eine Mischung von Ethylcellulose und Hydroxypropylmethylcellulose als verzögert freisetzendes Polymer enthält,
auf.

Multipartikulär im Sinne dieser Erfindung ist jede pharmazeutische Zusammensetzung, die in Form von Partikeln kleiner als 2 mm, vorzugsweise kleiner als 1,5 mm vorliegt oder kurz nach deren Einnahme, d.h. innerhalb von 30 min, vorzugsweise 15 min, in Partikel dieser Größe zerfällt. Insbesondere handelt es sich bei diesen Partikeln um Agglomerate von annähernd kugelförmiger oder zylindrischer Gestalt, deren mittlerer Durchmesser kleiner als.2 mm, vorzugsweise kleiner als 1,5 mm ist und besonders bevorzugt in einem Bereich von 0,1 bis 2 mm liegt. Im Gegensatz dazu sind monolithische Zusammensetzungen nach der Einnahme, zumindest bis zum Verlassen des Magens, größer als 2 mm.

Die Komponente (a1) der erfindungsgemäßen Zusammensetzung ist ein tri- oder tetracyclisches Antidepressivum und vorzugsweise Amitriptylin. Das Antidepressivum und insbesondere Amitriptylin kann in der Form der freien Base oder in Form eines pharmazeutisch annehmbares Salzes oder Solvats vorliegen. Beispiele von nützlichen Salzen von Amitriptylin und Amitriptylinoxid sind Chlorid, Bromid und Hydrogensulfat (Additionssalze mit einer anorganischen Säure) sowie Oxalat und Maleat (Additionssalze mit einer organischen Säure).

Vorzugsweise enthält die erfindungsgemäße Zusammensetzung Amitriptylin, Amitriptylinoxid, Amitriptylin-HCl oder Amitriptylinoxid-HCl und besonders bevorzugt Amitriptylin-HCl als Komponente (a1).

Des Weiteren ist es bevorzugt, dass die Zusammensetzung 5 bis 75 Gew.-%, insbesondere 10 bis 50 Gew.-% und besonders bevorzugt 20 bis 30 Gew.-% der Komponente (a1) enthält, bezogen auf das Gewicht der Zusammensetzung.

Die Komponente (a2) der erfindungsgemäßen Zusammensetzung ist ein Bindemittel, das ausgewählt ist aus Hydroxypropylmethylcellulose, Hydroxypropylcellulose, Polyvinylpyrrolidon, Polyvinylalkohol, Methylcellulose, Natriumcarboxymethylcellulose oder Mischungen davon. Es wurde gefunden, dass die Verwendung derartiger Bindemittel in multipartikulären Formulierungen von Amitriptylinzusammensetzungen mit erhöhter Lagerstabilität hinsichtlich dem chemischen Abbau des Wirkstoffs und seiner Freisetzung führt.

Ein bevorzugtes Bindemittel (a2) ist Hydroxypropylmethylcellulose (HPMC). Besonders vorteilhaft ist Hydroxypropylmethylcellulose vom Substitütionstyp 2910 (gemäß USP). Beispiele für Handelsnamen von nützlicher HPMC sind Pharmacoat 603, 606 oder 615.

Die Zusammensetzung enthält vorzugsweise 0,1 bis 10 Gew.-%, insbesondere 0,2 bis 5 Gew.-% und besonders bevorzugt 0,5 bis 2 Gew.-% des Bindemittels (a2), bezogen auf das Gewicht der Zusammensetzung.

Die Teilchen (a) der Zusammensetzung können auch mindestens einen weiteren Hilfsstoff enthalten.

Ein bevorzugter weiterer Hilfsstoff ist ein Stabilisator (a3). Geeignete Stabilisatoren sind Ascorbinsäure und deren Salze, Tocopherole sowie andere bekannte Antioxidantien. Besonders. bevorzugt enthalten die Teilchen (a) Ascorbinsäure als Stabilisator (a3).

Es ist bevorzugt, dass die erfindungsgemäße Zusammensetzung 0,5 bis 20 Gew.-%, insbesondere 1 bis 10 Gew.-% und besonders bevorzugt 2 bis 6 Gew.-% des Stabilisators (a3) enthält, bezogen auf das Gewicht der Zusammensetzung.

Das Gewichtsverhältnis der Komponente (a1), bezogen auf das freie Amitriptylin, zu Komponente (a3) ist vorzugsweise 2:1 bis 20:1, insbesondere 5:1 bis 10:1.

Ferner können die Teilchen (a) als weiteren Hilfsstoff ein Trennmittel (a4) enthalten. Insbesondere ist das Trennmittel ausgewählt aus der Gruppe bestehend aus Talkum, Siliciumdioxid, Magnesiumstearat und Mischungen davon.

Die Teilchen (a) sind vorzugsweise isometrisch und haben eine kugelige, zylindrische oder ähnliche Form. In einer bevorzugten Ausführungsform werden die Teilchen (a) gebildet, indem inerte Kerne, d.h. Starterkerne aus bekannten Arzneimittelhilfsstoffen, mit den Komponenten (a1), (a2) und ggf. weiteren Komponenten wie (a3) und (a4) beschichtet werden. Somit enthalten die Teilchen (a) in dieser Ausführungsform als weiteren Hilfsstoff inerte Kerne (a5), auf denen die übrigen Komponenten der Teilchen (a) aufgebracht sind. Brauchbare inerte Kerne sind bekannt und umfassen beispielsweise Kerne, die mikrokristalline Cellulose (MCC), Saccharose, Stärke oder Mischungen davon enthalten. Die Verwendung von inerten Kernen, die mikrokristalline Cellulose enthalten, ist besonders bevorzugt. Am meisten bevorzugt ist, dass die inerten Kerne im wesentlichen aus mikrokristalliner Cellulose bestehen. Besonders vorteilhaft ist die Verwendung von mikrokristalliner Cellulose mit einer mittleren Teilchengröße von 500 bis 700 µm.

Außerdem ist bevorzugt, dass die Zusammensetzung 10 bis 60 Gew.-%, insbesondere 20 bis 50 Gew.-% und besonders bevorzugt 30 bis 40 Gew.-% inerte Kerne enthält, bezogen auf das Gewicht der Zusammensetzung.

Alternativ zu der Verwendung von inerten Kernen, können die Teilchen (a) in einer anderen Ausführungsform gebildet werden, indem die Komponenten (a1), (a2) und ggf. weitere Komponenten z.B. durch Direktpelletieren oder durch Extrudieren und anschließendes Späronisieren in Teilchenform gebracht werden.

Die Komponente (b) der erfindungsgemäßen Zusammensetzung ist eine Schicht eines verzögert freisetzenden Polymers auf den Teilchen (a), das eine Mischung aus Ethylcellulose und Hydroxypropylmethylcellulose enthält.

Vorzugsweise beträgt das Gewichtsverhältnis von Ethylcellulose zu Hydroxypropylmethylcellulose in dieser Mischung 0,5:1 bis 20:1, insbesondere 3:1 bis 10:1 und besonders bevorzugt 6:1 bis 7:1.

Insbesondere kann die gleiche Hydroxypropylmethylcellulose verwendet werden wie sie unter (a2) beschrieben wird.

Des Weiteren kann die Schicht (b) mindestens einen weiteren Hilfsstoff enthalten.

So kann die Schicht (b) zur Verbesserung der Filmbildung und ihrer Flexibilität einen Weichmacher als weiteren Hilfsstoff enthalten. Geeignete Weichmacher sind Dibutylsebacat, Dibutylphthalat, 1,2-Propylenglycol, Polyethylenglycol, Triethylcitrat und Triacetin, wobei Dibutylsebacat der bevorzugte Weichmacher ist.

Außerdem kann die Schicht (b) als weiteren Hilfsstoff ein Trennmittel, wie z.B. Talkum oder Siliciumdioxid, enthalten.

Die Zusammensetzung kann weiterhin vorteilhafterweise zwischen den Teilchen (a) und der Schicht (b) mindestens eine Trennschicht (c) aufweisen. Diese Trennschicht enthält vorzugsweise einen Filmbildner, d.h. ein Polymer ausgewählt aus der Gruppe bestehend aus Hydroxypropylcellulose, Hydroxypropylmethylcellulose, Polyvinylpyrrolidon, Polyvinylalkohol, Methylcellulose, Natriumcarboxymethylcellulose und Mischungen davon, sowie ein Trennmittel wie Talkum.

Weiterhin kann auf der Schicht (b) vorteilhafterweise ein zusätzlicher Filmüberzug aufgebracht werden. Dieser abschließende Filmüberzug enthält vorzugsweise die gleichen Polymere wie die Trennschicht (c), sowie ein Trennmittel wie Talkum.

Die erfindungsgemäße multipartikuläre Zusammensetzung liegt vorzugsweise in Form von Pellets oder Granulaten und insbesondere in Form von Pellets vor. Pellets sind isometrische Agglomerate von Pulverpartikeln und weisen insbesondere die oben beschriebene Partikelgröße und -form auf. Typischerweise ist die Oberfläche der Pellets glatt und wenig porös. In einer bevorzugten Ausführungsform liegen die Pellets innerhalb einer Kapselhülle vor, die sich im Magen rasch auflöst.

Die Erfindung betrifft somit insbesondere auch mit den Pellets gefüllte Kapseln.

Es wurde gefunden, dass erfindungsgemäße Zusammensetzungen auch nach großer Lagerdauer ein gegenüber dem Ausgangswert kaum verändertes Wirkstofffreisetzungsprofil aufweisen. Vorzugsweise beträgt die Menge an freigesetztem Amitriptylin nach 1 Stunde höchstens 50%, nach vier Stunden zwischen 20 und 80% und nach 12 Stunden mindestens 70% der theoretisch möglichen Menge, bezogen auf den in Beispiel 4 angegebenen *in vitro-*Wirkstofffreisetzungstest.

Die Erfindung betrifft weiterhin ein Verfahren zur Herstellung von erfindungsgemäßen pharmazeutischen Zusammensetzungen, bei dem
(i) eine Lösung des Bindemittels (a2) bereitgestellt wird,
(ii) die Lösung aus Schritt (i) zusammen mit der Komponente (a1) und ggf. weiteren Hilfsstoffen (a3) und (a4) in eine Teilchenform umgewandelt wird und
(iii) die in Schritt (ii) erhaltenen Teilchen mit einer Schicht (b), die eine Mischung von Ethylcellulose und Hydroxypropylmethylcellulose als verzögert freisetzendes Polymer enthält, beschichtet werden.

In Schritt (i) wird das Bindemittel vorzugsweise in Ethanol gelöst.

Des Weiteren ist ein Verfahren bevorzugt, bei dem in Schritt (ii)
(ii') die Lösung aus Schritt (i) zunächst auf inerte Kerne (a5) gesprüht wird,
(ii'') anschließend Komponente (a1), ggf. als Mischung mit Hilfsstoffen (a3) und/oder (a4), bis zur Trocknung aufgetragen wird und
(ii''') die Schritte (ii') und (ii") so oft wiederholt werden, bis die gewünschte Menge an Komponente (a1) aufgetragen ist.

Allgemein stehen zur Herstellung der erfindungsgemäßen Zusammensetzung viele in der Technik bekannte Herstellungsverfahren und übliche Geräte wie Dragierkessel, Pelletierteller, Wirbelschichtgeräte oder Rotor-Wirbelschichtanlagen zur Verfügung.

Die Erfindung wird unter Bezug auf die folgenden Beispiele weiter veranschaulicht.

### Beispiele

### Beispiele 1-3: Herstellung von mit verzögert freisetzenden Amitriptylin-HCl-Pellets gefüllten Kapseln

Zur Herstellung der in Tabelle 1 angegebenen Pelletzusammensetzungen wurden die inerten Kerne in einem Dragierkessel vorgelegt und mit einer ethanolischen Lösung des Bindemittels für den Pelletkern besprüht. Anschließend wurde eine Mischung des Wirkstoffs mit Ascorbinsäure und Talkum bis zur Trockne auf die inerten Kerne aufgetragen. Die Auftragung der Bindemittellösung und der Wirkstoffmischung wurde abwechselnd so oft wiederholt, bis die gesamte erwünschte Wirkstoffmenge aufgetragen war. Die resultierenden Pellets wurden im Dragierkessel auf eine Restfeuchte von weniger als 3 Gew.-% nachgetrocknet und mit einem Sieb auf eine Kornklasse von 0,40 bis 1,25 mm klassiert.

In Beispiel 2 wurde dann die Trennschicht in einem Wirbelschichtgerät oder Dragierkessel aufgetragen, wobei der Filmbildner in Ethanol gelöst wurde, bevor das Trennmittel eingearbeitet wurde.

Zur Auftragung der Retardschicht wurde in den Beispielen 1 und 3 der Filmbildner in einer geeigneten Menge Ethanol gelöst. Das in Beispiel 2 verwendete Polyvinylacetat lag bereits als wässrige Dispersion vor (Kollicoat SR 30D). Das/die Trennmittel und der Weichmacher wurden zu den gelösten bzw. dispergierten Filmbildhern gegeben und die Retardschicht wurde in einem Wirbelschichtgerät oder einem Dragierkessel aufgetragen. Dann wurden die Pellets im Beschichtungsgerät nachgetrocknet und mit einem geeigneten Sieb auf eine Korngröße zwischen 0,5 und 1,4 mm klassifiziert.

Abschließend wurden die erhaltenen Pellets in handelsübliche Hartgelatinekapseln der Größe 2 (Beispiel 1 und 3) bzw. Größe 1 (Beispiel 2) abgefüllt.

Die Formulierungen können auch im industriellen Produktionsmaßstab (ca. 100 kg Pellets pro Charge) hergestellt werden.

**Tabelle 1: Zusammensetzungen der Beispielsformulierungen**

| **Komponente** | **Funktion** | **Bsp. 1*** (mg) | **Bsp. 2*** (mg) | **Bsp. 3** (mg) |
|---|---|---|---|---|
| **Pelletkern** | | | | |
| Amitriptylin-HCl | Wirkstoff | 75,00 | 75,00 | 75,00 |
| Ascorbinsäure | Stabilisator | 11,25 | 11,25 | 11,25 |
| Zucker/Stärke-Pellets | inerte Kerne | 117,51 | - | - |
| MCC-Pellets | inerte Kerne | - | 100,00 | 100,00 |
| Schellack | Bindemittel | 6,57 | - | - |
| HPMC | Bindemittel | - | 2,56 | 2,56 |
| Talkum | Trennmittel | 39,72 | 65,41 | 61,19 |
| **Trennschicht** | | | | |
| HPC | Filmbildner | - | 2,75 | - |
| Talkum | Trennmittel | - | 1,38 | - |
| **Retardschicht** | | | | |
| Schellack | Filmbildner | 14,94 | - | - |
| Triethylcitrat | Weichmacher | 0,75 | - | - |
| Talkum | Trennmittel | 10,47 | 33,05 | 15,00 |
| Ethylcellulose | Filmbildner | - | - | 15,00 |
| HPMC | Filmbildner | - | - | 2,25 |
| Dibutylsebacat | Weichmacher | - | - | 2,25 |
| Polyvinylacetat | Filmbildner | - | 66,10 | - |
| Polyvinylpyrrolidon | Filmbildner | - | 10,58 | - |
| Propylenglycol | Weichmacher | - | 6,61 | - |
| Siliciumdioxid | Trennmittel | - | 7,93 | - |
| **Füllgewicht pro Kapsel** | | 276,14 | 382,62 | 284,50 |

| | | | | |
|---|---|---|---|---|
| *: Vergleichsbeispiel | | | | |

### Beispiel 4: Lagerstabilität

Ein Teil der in den Beispielen 1 bis 3 erhaltenen Kapseln wurde direkt nach der Herstellung auf die Reinheit des Wirkstoffs und die Wirkstofffreisetzung hin untersucht.

Die Reinheit von Amitriptylin wurde mittels HPLC unter Verwendung eines Alliance 2695 von Waters mit folgenden Parametern bestimmt:

| | |
|---|---|
| Säule: | RP 18, Symmetrie Shield; 5 µm; 3,9 x 150 mm ID; |
| Eluent: | Dikaliumhydrogenphosphatpuffer 0,01 mol/l : Methanol (HPLC Qualität) 1:0,89 (m/m); eingestellt auf pH 7,5; |
| Fluss: | 1,4 ml/min; |
| Injektionsvolumen: | 20 µl; |
| Analysenzeit: | 25 Minuten; |
| Detektor: | 2996 PDA; |
| Messwellenlänge: | 238 nm (Gehalt Amitriptylin und Cycloben-zaprin) 273 nm (Gehalt Dibenzosuberon); |
| Temperatur: | 35 °C. |

Zur Bestimmung des Freisetzungsprofils wurde in einer paddle-Apparatur bei 100 U/min ein in vitro-Freisetzungstest durchgeführt, wobei 900 ml einer Pufferlösung mit pH 1,5 als Freisetzungsmedium verwendet wurden. Zur Bestimmung des freigesetzten Wirkstoffs wurde ein Alliance 2695 von Waters mit folgenden Parametern verwendet:

| | |
|---|---|
| Säule: | RP 18, Symmetrie Shield; 5 µm; 3,9 x 150 mm ID; |
| Eluent: | Dikaliumhydrogenphosphatpuffer 0,01 mol/l : Methanol (HPLC Qualität) 1:0,89 (m/m); eingestellt auf pH 3,5; |
| Fluss: | 1,8 ml/min; |
| Injektionsvolumen: | 20 µl; |
| Analysenzeit: | ca. 5 Minuten; |
| Detektor: | 2996 PDA; |
| Messwellenlänge: | 238 nm; |
| Temperatur: | 35 °C. |

Ein weiterer Teil der in den Beispielen 1 bis 3 hergestellten Kapseln wurde bei verschiedenen Temperaturen und relativen Feuchtigkeiten in PVC/PVdC/COC-Alu-Blistern (Pentapharm SD03) im Dunkeln gelagert. Nach einer definierten Lagerzeit wurden die Zusammensetzungen dann nach den obigen Verfahren auf die vorhandenen Verunreinigungen des Wirkstoffs und das Freisetzungsprofil hin untersucht.

Die Ergebnisse der obigen Untersuchungen sind in den Tabellen 2 und 3 dargestellt.

**Tabelle 2: Untersuchung der Reinheit (Angabe in Gew.-%)**

| **Lagerbedingungen** | **Verunreinigungen sowie Aussehen** (Spezifikationen in Klammern) | **Bsp. 1** | **Bsp. 2** | **Bsp. 3** |
|---|---|---|---|---|
| Ausgangswert | Cyclobenzaprin (max. 0,2%) | n.n. | n.n. | n.n. |
| | Dibenzosuberon (max. 0,2%) | n.n. | n.n. | 0,02% |
| | Unbekannte Verunr. Einzeln (max. 0,2%) | entspr. | entspr. | entspr. |
| | Unbekannte Verunr. Summe (max. 0,5%) | 0,05% | 0,05% | 0,08% |
| | Aussehen (weiß bis gelblich) | entspr. | entspr. | entspr. |
| 25°C, 60% r.F., 3 Monate | Cyclobenzaprin (max. 0,2%) | 0,02% | 0,02% | 0.03%- |
| | Dibenzosuberon (max. 0,2%) | 0,18% | n.n. | 0,04% |
| | Unbekannte Verunr. Einzeln (max. 0,2%) | entspr. | entspr. | entspr. |
| | Unbekannte Verunr. Summe (max. 0,5%) | 0,44% | 0,06% | 0.21% |
| | Aussehen (weiß bis gelblich) | hell-braun | entspr. | entspr. |
| 30°C, 65% r.F., 3 Monate | Cyclobenzaprin (max. 0,2%) | 0,02% | 0,02% | 0.03%- |
| | Dibenzosuberon (max. 0,2%) | 0,15% | n.n. | 0,04% |
| | Unbekannte Verunr. Einzeln (max. 0,2%) | entspr. | entspr. | entspr. |
| | Unbekannte Verunr. Summe (max. 0,5%) | 0,36% | 0,09% | 0.22% |
| | Aussehen (weiß bis gelblich) | entspr. | entspr. | entspr. |
| 40°C, 75% r.F., 3 Monate | Cyclobenzaprin (max. 0,2%) | - | 0,02% | 0.04%- |
| | Dibenzosuberon (max. 0,2%) | - | 0,06% | 0,07% |
| | Unbekannte Verunr. Einzeln (max. 0,2%) | - | entspr. | entspr. |
| | Unbekannte Verunr. Summe (max. 0,5%) | - | 0,11% | 0.18% |
| | Aussehen (weiß bis gelblich) | - | entspr. | entspr. |

Wie aus Tabelle 2 ersichtlich ist, weisen die erfindungsgemäßen Zusammensetzungen eine hohe Lagerstabilität hinsichtlich der Wirkstoffreinheit auf. Die Mengen an Verunreinigungen liegen bei allen Lagerbedingungen eindeutig unterhalb der vorgeschriebenen Grenzwerte. Auch die Vergleichsprobe aus Beispiel 1 ist in Bezug auf den Wirkstoffabbau formal als lagerstabil zu beurteilen. Allerdings treten für diese Zusammensetzung schon nach 3 Monaten bei einer Lagerung bei 25°C und 60% r.F. eine Änderung des Aussehens auf und die Menge der Summe der unbekannten Verunreinigungen liegt mit 0,44% nur knapp unter dem Grenzwert von 0,5%.

Gegenüber den am Markt befindlichen Zusammensetzungen in Form von Hydrokolloidmatrixtabletten sind die erfindungsgemäßen multipartikulären Formulierungen als viel zufriederistellender zu bezeichnen (vgl. z.B. Saroten® retard Tabs 75 mg, Charge: BXBBCJ2, 3 Monate Lagerung bei 40°C und 75% r.F. in Polypropylendosen: unbekannte Verunreinigung bei RRT 0,57 in einer Menge von 0,38% und Summe der unbekannten Verunreinigungen: 0,78%)

**Tabelle 3: Untersuchung des Freisetzungsprofils**

| **Lagerbedingungen** | **Zeit Freisetzungstest** (Spezifikation der freigesetzten Menge in Klammern) | **Menge an freigesetztem Amitriptylin (Gew.-%)** | | |
|---|---|---|---|---|
| | | **Bsp. 1** | **Bsp. 2** | **Bsp. 3** |
| Ausgangswert | 1h (<50%) | 22,9 | 14,1 | 22,0 |
| | 4h (20-80%) | 80,0 | 60,4 | 59,0 |
| | 12h (>70%) | 89,5 | 81,1 | 82,0 |
| 25°C, 60% r.F., 3 Monate | 1h (<50%) | 92,6 | 16,4 | 23,0 |
| | 4h (20-80%) | 96,5 | 54,1 | 59,0 |
| | 12h (>70%) | 98,5 | 83,3 | 82,0 |
| 30°C, 65% r.F., 3 Monate | 1h (<50%) | 89,2 | 15,0 | 24,0 |
| | 4h (20-80%) | 94,3 | 28,6 | 60,0 |
| | 12h (>70%) | 95,8 | 81,9 | 81,0 |
| 40°C, 75% r.F., 3 Monate | 1h (<50%) | - | 13,5 | 24,0 |
| | 4h (20-80%) | - | 16,9 | 57,0 |
| | 12h (>70%) | - | 63,7 | 79,0 |

Wie aus Tabelle 3 erkannt werden kann, sind die Formulierungen aus Beispiel 1 und 2 in Bezug auf die Freisetzung des Wirkstoffs nicht lagerstabil. Bei Formulierungen gemäß Beispiel 1 werden bereits nach einer Lagerung von 3 Monaten bei 25°C und 60% r.F. 92,6% des Amitriptylins innerhalb von einer Stunde freigesetzt, während direkt nach der Herstellung der Pellets in der ersten Stunde nur 22,9 % freigesetzt wurden. Bei Formulierungen gemäß Beispiel 2 werden nach einer Lagerung von 3 Monaten bei 40 °C und 75 % r.F. die vorgeschriebenen Freisetzungsmengen nach 4 und 12 h deutlich unterschritten. Im Gegensatz dazu zeigen die erfindungsgemäßen Zusammensetzungen aus Beispiel 3, bei der die Retardschicht Ethylcellulose und Hydroxypropylmethylcellulose enthält, ein wünschenswertes Freisetzungsprofil von Amitriptylin, das selbst bei einer Lagerung bei 40°C und 75% r.F. annähernd konstant ist.

## Patentansprüche

1. Multipartikuläre pharmazeutische Zusammensetzung, die
(a) Teilchen, die
(a1) ein tri- oder tetracyclisches Antidepressivum oder ein pharmazeutisch annehmbares Salz oder Solvat davon und
(a2) ein Bindemittel ausgewählt aus Hydroxypropylmethylcellulose, Hydroxypropylcellulose, Polyvinylpyrrolidon, Polyvinylalkohol, Methylcellulose, Natriumcarboxymethylcellulose oder Mischungen davon
enthalten, und
(b) eine Schicht auf den Teilchen (a), welche eine Mischung von Ethylcellulose und Hydroxypropylmethylcellulose als verzögert freisetzendes Polymer enthält,
aufweist.

2. Zusammensetzung nach Anspruch 1, die Amitriptylin, Amitriptylinoxid, Amitriptylin-HC1 oder Amitriptylinoxid-HCl und vorzugsweise Amitriptylin-HC1 als Komponente (a1) enthält.

3. Zusammensetzung nach Anspruch 1 oder 2, die 5 bis 75 Gew.-% der Komponente (a1) enthält.

4. Zusammensetzung nach einem der Ansprüche 1 bis 3, die Hydroxypropylmethylcellulose als Bindemittel (a2) enthält.

5. Zusammensetzung nach einem der Ansprüche 1 bis 4, die 0,1 bis 10 Gew.-% des Bindemittels (a2) enthält.

6. Zusammensetzung nach einem der Ansprüche 1 bis 5, bei der die Teilchen (a) mindestens einen weiteren Hilfsstoff enthalten.

7. Zusammensetzung nach Anspruch 6, die als weiteren Hilfsstoff einen Stabilisator (a3) ausgewählt aus Ascorbinsäure, Tocopherolen oder Mischungen davon enthält.

8. Zusammensetzung nach Anspruch 7, die 0,5 bis 20 Gew.-% des Stabilisators (a3) enthält.

9. Zusammensetzung nach Anspruch 6, die als weiteren Hilfsstoff ein Trennmittel (a4) ausgewählt aus Talkum, Siliciumdioxid, Magnesiumstearat oder Mischungen davon enthält.

10. Zusammensetzung nach einem der Ansprüche 1 bis 9, bei der die Teilchen (a) inerte Kerne (a5) aufweisen, auf denen die übrigen Komponenten der Teilchen (a) aufgebracht sind.

11. Zusammensetzung nach Anspruch 11, bei der die inerten Kerne (a5) mikrokristalline Cellulose, Saccharose, Stärke oder Mischungen davon und vorzugsweise mikrokristalline Cellulose enthalten.

12. Zusammensetzung nach Anspruch 10 oder 11, die 10 bis 60 Gew.-% inerte Kerne enthält.

13. Zusammensetzung nach einem der Ansprüche 1 bis 12, bei der das Gewichtsverhältnis von Ethylcellulose zu Hydroxypropylmethylcellulose in Schicht (b) 0,5:1 bis 20:1 beträgt.

14. Zusammensetzung nach einem der Ansprüche 1 bis 13, bei der die Schicht (b) mindestens einen weiteren Hilfsstoff enthält.

15. Zusammensetzung nach Anspruch 14, bei der der weitere Hilfsstoff ein Weichmacher ist.

16. Zusammensetzung nach Anspruch 14 oder 15, die als weiteren Hilfsstoff in Schicht (b) ein Trennmittel enthält.

17. Zusammensetzung nach einem der Ansprüche 1 bis 16, bei der zwischen den Teilchen (a) und der Schicht (b) mindestens eine Trennschicht (c) vorhanden ist.

18. Zusammensetzung nach einem der Ansprüche 1 bis 17, die in Form von Pellets oder Granulaten und vorzugsweise in Form von Pellets vorliegt.

19. Zusammensetzung nach Anspruch 18, bei der die Pellets innerhalb einer Kapselhülle vorliegen.

20. Zusammensetzung nach einem der Ansprüche 1 bis 22, bei der die Menge an freigesetztem Antidepressivum nach 1 Stunde höchstens 50%, nach vier Stunden zwischen 20 und 80% und nach 12 Stunden mindestens 70% der theoretisch möglichen Menge beträgt, bezogen auf den in vitro-Freisetzungstest gemäß Beispiel 4.

21. Verfahren zur Herstellung einer pharmazeutischen Zusammensetzung gemäß einem der Ansprüche 1 bis 20, bei dem
(i) eine Lösung des Bindemittels (a2) bereitgestellt wird,
(ii) die Lösung aus Schritt (i) zusammen mit der Komponente (a1) und ggf. weiteren Hilfsstoffen (a3) und (a4) in eine Teilchenform umgewandelt wird und
(iii) die in Schritt (ii) erhaltenen Teilchen mit einer Schicht (b), die eine Mischung von Ethylcellulose und Hydroxypropylmethylcellulose als verzögert freisetzendes Polymer enthält, beschichtet werden.

22. Verfahren nach Anspruch 21, bei dem in Schritt (i) das Bindemittel in Ethanol gelöst wird.
